(19) Europäisches Patentamt
European Patent Office
Office européen des brevets

(11) Publication number: **0 430 032 A1**

(12) # EUROPEAN PATENT APPLICATION

(21) Application number: **90122173.9**

(51) Int. Cl.[5]: **A61K 7/13**, D06P 1/642

(22) Date of filing: **20.11.90**

(30) Priority: **22.11.89 US 440529**

(43) Date of publication of application:
**05.06.91 Bulletin 91/23**

(84) Designated Contracting States:
**DE FR GB SE Bulletin**

(71) Applicant: **Bristol-Myers Squibb Company**
**345 Park Avenue**
**New York, N.Y. 10022(US)**

(72) Inventor: **Wenke, Gottfried**
**Six Cowpath Lane**
**Woodbridge Connecticut 06525(US)**

(74) Representative: **Graalfs, Edo, Dipl.-Ing. et al**
**Neuer Wall 41**
**W-2000 Hamburg 36(DE)**

(54) **Dye couplers.**

(57) A composition for coloring keratinaceous fibers comprising, as coupler, one or more compounds of formula I:

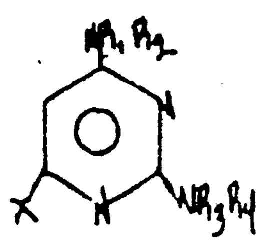

(I)

wherein X is an $NH_2$, SH, or OH group, and each of $R_1$ through $R_4$ is independently selected from H and $C_{1-6}$ alkyl moieties.

EP 0 430 032 A1

# DYE COUPLERS

## FIELD OF THE INVENTION

This invention is directed to oxidation hair dyes. More specifically, this invention is directed to the use of trisubstituted pyrimidines as coupling components in oxidation hair dyes.

## BACKGROUND

The oxidative, or permanent, dyeing of keratinaceous substrates, eg., human hair, generally involves the use of a coupler component, a developer, or primary intermediate, component and an oxidizer. The developer (or primary intermediate) is oxidized by the oxidizing agent during the dyeing process and serves as the component which gives the hair the basic (ie., background) color--usually browns of varying intensity.

The couplers, in contrast, are not oxidized during dyeing. Rather, they react with the (oxidized) developer to give the hair the final color, in particular, impart fashionable color highlights. Para-phenylene diamine derivatives, diaminopyridines,4-aminopyrazolone derivatives, and tetraOand triamino-pyrimidines are useful developers. Phenols, m-phenylene diamine derivatives, naphthols, resorcinol derivatives and pyrazolones are useful as coupling components.

While many color combinations can be obtained by the developers and couplers, known in the art, there is a lack of couplers which produce bright yellow or bright red colors. To compensate for this, direct dyes are sometimes added to oxidation dye products. This, however, sometimes leads to off-shade fading, since the direct dyes cannot match the good wearing properties, eg., washfastness, of oxidation dyes.

A disadvantage of the oxidation dyes known in the prior art is the difficulty of removing them from hair, if the resultant dyeing is undesired. It has been reported, that some oxidation dyes are removed with relative ease by a reductive treatment. However, since the cystine linkages in hair are also easily reduced, the hair is left in poor condition after such treatment. Therefore, it would be desirable, if oxidation dyes could be removed under non-reducing conditions.

## THE INVENTION

It has been discovered that compounds of formula I

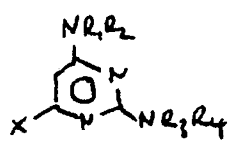

(I)

wherein X = $NH_2$, SH or OH, and each of $R_1$ through $R_4$ is independently selected from H and $C_1$-6 alkylmoieties, are useful as couplers oxidation dyeing systems.

In preferred embodiments, 2,4,6-triaminopyrimidines and 2, and 2,4-diamino-6-hydroxy-pyrimidine give bright yellow dyeouts, in one percent concentration, with 0.5% tetraaminopyrimidine as developer and 3% $H_2O_2$ oxidant.

The invention is directed to coloring compositions containing the compounds discussed above and to methods of coloring keratinaceous substrates therewith.

The triamino pyrimidines and 2,4-diamino-6-hydroxy pyrimidines to be used as coupline components according to the invention, are themselves well known compounds.

U.S. 3,325,496 describes synthesis and use of 2,4,6-triaminopyrimidines in lubricants and other functional fluids, based on their viscosity/temperature properties. The synthesis of 2,4-diamino-6-hydroxy-pyrimidine from guanidine hydrochloride and cyanoacetic ester has been reported (Traube, Ber. 33,1371,-(1900).

Pyrimidine derivatives are know to be useful as developers in the art of hair dyeing.

U.S. 4,046,503 discloses water-soluble 2,4,5-triaminopyrimidine-6-($C_{1-4}$ alkoxy) pyrimidines as developers in oxidation dyeing systems. The pyrimidines were selected as developers to overcome the toxicological and dermatological problems associated with p-phenylenediamine developers.

U.S. 4,129,413 reveals oxidation hair dyes which contain 2,4,5,6-tetramino pyrimidine as a developer with alkyl-m-dihydroxybenzene couplers.

U.S. 4,168,953 is concerned with oxidation hair dyes which employ tetraamino pyrimidines as developers of hydroxindazole couplers.

U.S. 4,213,758 deals with tetraamino pyrimidines as developers for a variety of couplers containing amino-, chloro-, hydroxy-, and/or alkoxy-substituted aryl groups.

U.S. Re. 30,199 (Reissue of 4,003,699) describes tetraaminopyrimidines as developers for phenylene-based couplers.

German Offen. 2,523,045 relates to oxidation dyeing systems in which the developer component is an oxo- or thioxo-pyrimidine bearing two amino substituents.

German Offen. 2, 524,329 shows that dioxo- and trioxo-pyrimidines are useful as developers for oxidative dyeing systems.

German Offen. 2,932,800, deals with tetra-substituted aminopyrimidines as developers with amino-benzo-thiadiazole couplers.

The use of pyrimidine derivatives as couplers is new. It is also unexpected because of the character of the pyrimidine ring. The electron withdrawing effect of two hetero-nitrogens would cause one to expect low electron density--and, therefore, little reactivity at the coupling position on the ring.

Equally unexpected are the colors, which are obtained after the coupling reaction, when the new pyrimidine couplers are compared to well known couplers, in particular m-diaminobenzenes and m-amino-hydroxybenzenes.

For example, 2,4,6-triaminopyrimidine and 2-chloro-p-phenylenediamine impart a golden yellow shade to hair. In contrast, the known m-diaminobenzene couplers with p-phenylene-diamine derivatives usually give blue colors. With 2-chloro-p-phenylene diamine and m-phenylene diamine, a dull gray color was imparted to hair.

Finally, it was founded that color highlights imparted to hair by the new pyrimidine couplers are removed from hair to a large extent by a short treatment with alkaline peroxide. This is unexpected, since it is not observed with the known oxidation dyes.

## ADVANTAGES

The invention has a number of advantages over other coloring systems for keratinaceous fibers.

The fibers can be permanently dyed with colors which are otherwise difficult to obtain. While yellow shades can be produced using 2-methylresorcinol couplers with para-aminophenol developer and red shades can be obtained using 5-amino-orthocresol coupler with para-aminophenol developer, the resultant colors are different orange nuances, rather than true yellows or reds.

The use of 2,4,6-triaminopyrimidine and 2,4-diamino-6-hydroxypyrimidine couplers with tetraaminopyrimidine developer and 2-chloro-p-phenylenediamine developer to yield yellow and red gives much brighter colors.

The attainment of bright colors in an oxidation coloring system is important, because these colorings are more durable then those obtained from non-oxidation dyes, eg., direct dyes. The colors do not wash out as quickly as those obtain with direct dyes.

The compounds discussed herein can be used as couplers with various developers to produce a wide range of colors, including blues and brown-yellows.

Oxidation dyes frompyrimidine couplers can be removed from hair to a large extent by a short treatment with alkaline peroxide. Such treatment leaves the hair in good condition. If a hair color product is formulated with the exclusive use of pyrimidine couplers, the result of a dyeing process can be more easily corrected--or even reversed--than is possible with known oxidation dye products.

The dyeouts obtained using the invention exhibit good coverage of grey hair, good wearing properties and good color stability.

Furthermore, the couplers of the invention are water soluble and have good storage stability, making their packaging and handling easier.

These and other aspects and advantages of the invention will be aparent after consideration of the following description and claims.

## DESCRIPTION OF THE INVENTION

The invention will be described in terms of the coupling compounds, compositions and methods using same, and substrates treatable with same.

## COMPOUNDS

The compounds useful herein are employed in the "coupler" or "coupling agent" components of oxidative hair coloring systems. Such systems generally employ "developers" and "oxidants" or "oxidizing agents" as well.

The compounds used herein conform to formula I as described above. Preferred compounds are thos confirming to formula II as follows:

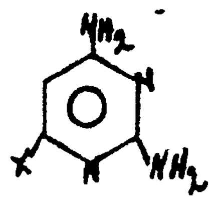

(II)

wherein X is $NH_2$, SH, or OH.

Highly preferred compounds are those of formula II in which X is $NH_2$ or OH.

Mixtures of compounds maybe used, as may the salts and other colorforming derivatives, eg., hydrochloric acid salts, sulfuric acid salts, and hydrates and the like.

The coloring systems of the invention may contain combinations of pyrimidine-based and non-pyrimidine-based couplers.

COMPOSITIONS

The compositions in which the couplers of the invention are used can contain--in addition to developer and oxidizer components which are usually employed--a variety of conventional ingredients. Among these are excipients (eg.,foaming agents, fillers, perfumes, processing aids and diluents) and functional additives (eg., buffers, stabilizers, pH modifiers, and the like). These additives are generally used in amounts appropriate to their functions in the final compositions.

Generally, such additives will be present in amounts ranging from about 0.5 to about 50 wt. %.

Unless otherwise stated, all percentages used herein are weight percentages based on total composition weight.

The amount of coupler component--ie., the total quantity of all couplers in the system--will be from about 0.005 to about 3 percent, with about 0.1 to about 2.5 percent preferred.

The amount of developer component (total of all developers) will be in the range of about 0.001 to about 2 percent, with about 0.005 to about 1 percent preferred.

The total oxidant component will be an amount within the range of about 1 to 6 percent.

The pH of the subject composition will generally be from about 6 to about 10, preferably to 6.5 to about 7.5, at the time that it is contacted with the substrate to be treated with the oxidative colorant(s).

Suitable pH modifiers are such reagents such as ammonia, alkylamines, alkanolamines, sodium and potassium hydroxides or carbonates, or, if necessary, acids, such as lactic, acetic, tartaric or phosphoric acid. Mixtures are operable.

The compositions of the invention can contain catalytic amounts of metal ions and/or other agents which assist the oxidant or otherwise facilitate the oxidative coupling of the two or more color-forming ingredients.

Suitable amounts of such agents--with copper and manganese being exemplary--are about 0.0001 to about 1 wt.%.

DEVELOPERS AND OXIDANTS

The developer and oxidant components of the instant compositions employ well known materials. Useful developers include tetra-aminopyrimidine, 2-chloro-p-phenylenediamine, p-phenylene diamine, N-methyl-p-phenylene diamine, N, N-bishydroxyethyl-p-phenylene-diamine, with tetraaminopyrimidine, 2-chloro-p-phenylene diamine and N-methyl-p-phenylene diamine preferred. Mixtures are operable.

Useful oxidants are well known oxidizing agents whose activity and physical properties make them suitable for incorporation into hair care products. Generally the oxidants used herein will be selected from hydrogen peroxide other inorganic peroxides, peroxo salts, such as sodium perborate or organic peroxides, such as urea peroxide. Preferred oxidants are hydrogen peroxide, sodium perborate and urea peroxide.

Mixtures are operable.

It is generally preferred that the ratio of coupler to developer be on the order of 0.1:1 to 20:1, with ratios of 1:1 to 10:1 preferred.

The quantity of oxygen is not critical. However, it is generally preferred that the oxidant be present in a sufficient amount so that its ratio to the developer is about 5:1 to about 50:1.

## FORMS OF COMPOSITIONS

The physical form of the instant compositions is not critical. They may comprise dry blends of the reagents used. However, for ease of mixing, application and/or handling, it is generally preferred that they be liquid or semi-solid, eg., gel- or paste-like.

The use of thickened gels or mousses can be employed to produce color effects in which only desired portions of the hair are colored, while others are left uncolored or colored differently.

When diluents are employed, they usually comprise suitable quantities of water or water-based co-diluent systems. Co-diluents may be ethanol, isopropanol and the like. Diluents amounts of 1 to 40 wt. % are contemplated.

## PROCESSES

The coloring system of the invention is used via the following general steps:

1. Contacting the substrate with the coupler, developer, and oxidant at the desired pH for about 5 to about 45 minutes, preferably about 10 to about 20 minutes:
2. Rinsing the substrate;
3. Shampooing, or otherwise washing, the substrate; and
4. Drying.

Optionally, step 1 may be preceded by a pretreatment step, in which metal ions or other catalysts are applied to the hair, followed by a water rinse.

Step (1) may be broken up into two or more substeps in which one or more each of these three reagents is contacted with the substrate separately. That is, the coupler may be applied first, followed by a mixture of the developer and oxidant. Alternatively, the coupler and developer may be premixed and applied before or after the oxidant. Other combinations of such substeps are contemplated.

The treatment of the substrate, eg., hair, colored in accordance with the invention may be preceded or followed by conventional chemical and/or physical hair treatment. Thus, perming, relaxing, conditioning, rolling, brushing, styling, pressing and the like can be carried out before--but preferably after--the coloring takes place.

## SUBSTRATES

Substrates to be treated with the system of the invention and oxidatively colored thereby will generally be of a keratinaceous nature. Accordingly, hair, fur, feathers, and the like may be colored. Substrates of animal, preferably mammalian, origin are preferred. Human hair is highly preferred.

By "hair" applicant means both living and non-living animal hair. Thus, "living" hair or hair growing from or on a living body is treatable, as is hair which has been cut or removed from a living body, eg., fur, wigs, or hair pieces.

## AUXILIARY COLORANTS

While the invention does not require the used of non-oxidative colorants, one or more of such pigments or dyes can be used as an auxiliary colorant. When used, these colorant's concentrations will be in minor amounts, ie., from about 0.001 to about 10%, based on total colorant content..

## EXAMPLES

The following examples illustrate the invention.

### Example 1

Dye compositions containing 1 wt. % of couplers, 0.5 wt. % of developer and 3 wt. % $H_2O_2$ in aqueous

solution was contacted with hair swastches of grey color.

The couplers and resultant dyeout colors for each dyeing are given below in Table I.

TABLE I: COLORS OBTAINED DURING OXIDATIVE DYEING

| NO. | COUPLER | COUPLER | DEVELOPER | COLOR |
|---|---|---|---|---|
| 1. | 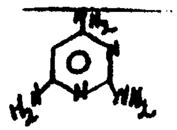 | 2,4,6-Triaminophyrimidine | 2-Chloro-p-phenylene diamine | Golden yellow |
| 2. | 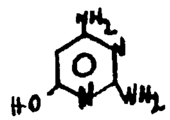 | 2,4-Diamino-6-hydroxypyrimidine | 2-Chloro-p-phenylene diamine | Red |
| 3. | 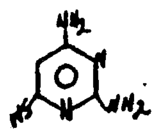 | 2,4-Diamino-6-mercaptopyrimidine | 2-Chloro-p-phenylene diamine | Brown yellow |
| 4. | 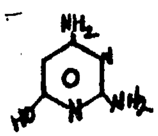 | 2, 4-Diamino-6-hydroxy pyrimidine | Tetraaminopyrimidine | Yellow |
| 5. | 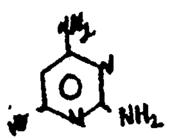 | 2,4-Diamino-6-hydroxy pyrimidine | N-methyl-p-phenylene diamine | Violet |
| 6. | | 2,4-Diamino-6-hydroxy pyrimidine | NN-bis-hydroxyethyl-p-phenylene diamine | Blue |

Hair, dyed violet according to No. 5, was exposed to 3% $H_2O_2$, pH10 for 3 minutes and rinsed with water. The hair showed the original grey color, which it had before dyeing.

## Claims

1. A composition for coloring keratinaceous fibers comprising, as coupler, one or more compounds of formula I:

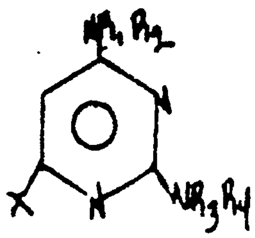

(I)

wherein X is an $NH_2$, SH, or OH group, and each of $R_1$ through $R_4$ is independently selected from H and $C_{1-6}$ alkyl moieties.
2. The composition of Claim 1 wherein H is $NH_2$ or OH.
3. The compositions of Claim 2 wherein the coupler is 2,4,6-triaminopyrimidine.
4. The composition of Claim 2 wherein the coupler is 2,4-diamino-6-hydroxypyrimidine.
5. The composition of Claim 1 further containing a developer.
6. The composition of Claim 5 wherein the developer is tetraamino-pyrimidine.
7. The composition of Claim 3 wherein the coupler is at least one compound selected from the group consisting of tetraaminopyrimidine, 2-chloro-p-phenylenediamine and N-methyl-p-phenylenediamine.
8. The composition of Claim 4 wherein the developer is at least one compound selected from the group consisting of tetraaminopyrimidine, 2-chloro-p-phenylenediamine and N-methyl-p-phenylenediamine.
9. The composition of Claim 1 wherein the coupler is present at a concentration of about 0.005 to about 3 wt.%.
10. The composition of Claim 5 wherein the developer is present at a concentration of about 0.001 to about 2 wt. %.
11. A process for the oxidative coloring of keratinaceous substrates comprising the step of contacting the substrate with a composition containing at least one compound of formula I:

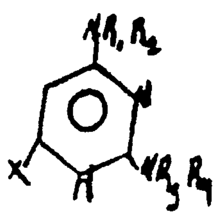

(I)

wherein X is an $NH_2$, SH, or OH group, and each of $R_1$ through $R_4$ is independently selected from H and $C_{1-6}$ alkyl moieties.
12. The process of Claim 11 wherein the coupler is one in which $R_1$ through $R_4$ are H.
13. The process of Claim 12 wherein the coupler is 2,4,6-triaminopyrimidine.
14. The process of Claim 12 wherein the coupler is 2,4-diamino-6-hydroxypyrimidine.
15. The process of Claim 11 wherein the composition also contains a developer.
16. The process of Claim 15 wherein the developer is at least one compound selected from he group consisting of tetraaminopyrimidine, 2-chloro-p-phenylenediamine and N-methyl-p-phenylenediamine.
17. A hair coloring kit containing, in one component, at least one compound of formula I:

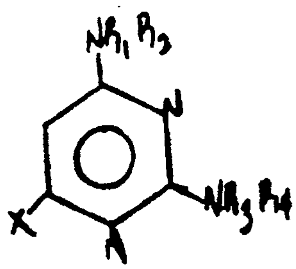

(I)

wherein X is anNH$_2$, SH, or OH group, and each of $R_1$ through $R_4$ is independently selected from H and $C_{1-6}$ alkyl moieties.

European Patent Office

# EUROPEAN SEARCH REPORT

Application Number

EP 90122173.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP - A2 - 0 299 497 (KAO CORP.) * Page 5, line 54 - page 7, line 29 * -- | 1-5,9, 15,17 | A 61 K 7/13<br>D 06 P 1/642 |
| P,X | EP - A2 - 0 376 078 (KAO CORP.) * Page 2, line 42 - page 3, line 51 * -- | 1-17 | |
| P,X | EP - A2 - 0 345 728 (KAO CORP.) * Page 3, line 1 - page 4, line 38 * ---- | 1-5, 9-15, 17 | |

TECHNICAL FIELDS SEARCHED (Int. Cl.5)

A 61 K 7/00
D 06 P 1/00
C 07 D 239/00

The present search report has been drawn up for all claims

| Place of search | Date of completion of the search | Examiner |
|---|---|---|
| VIENNA | 22-02-1991 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)